# EUROPEAN PATENT SPECIFICATION

(11) **EP 2 818 558 B1**
(45) Date of publication and mention of the grant of the patent: **17.07.2019**
(21) Application number: 13807284.8
(22) Date of filing: 21.05.2013
(51) Int. Cl.: C12Q 1/6806, C12Q 1/70

(54) **METHOD FOR ASSESSING THE VIABILITY OF VIRUSES WITH LYMPHOTROPISM**
VERFAHREN ZUR BEURTEILUNG DER LEBENSFÄHIGKEIT VON VIREN MIT LYMPHOTROPISMUS
PROCÉDÉ POUR ÉVALUER LA VITALITÉ DE VIRUS PRÉSENTANT UN LYMPHOTROPISME

(30) Priority: 18.06.2012 UZ 1200233
(43) Date of publication of application: 31.12.2014
(60) Divisional application: 19161185.4
(73) Proprietor: Mkrtchyan, Ovik Leonardovich, Tashkent 100187 (UZ)
(72) Inventor: GULYAMOV, Nariman, Tashkent 100027 (UZ)
(74) Representative: Diehl & Partner GbR
(86) International application number: PCT/UZ2013/000001
(87) International publication number: WO 2013/192636

(56) References cited:
- US-A1- 2009 176 202
- KOYA ARIYOSHI ET AL: "Development of a rapid quantitative assay for HIV-1 plasma infectious viraemia-culture-PCR (CPID)", JOURNAL OF MEDICAL VIROLOGY, vol. 43, no. 1, 1 May 1994 (1994-05-01), pages 28-32, XP055207266, ISSN: 0146-6615, DOI: 10.1002/jmv.1890430106
- S. PALMER ET AL: "New Real-Time Reverse Transcriptase-Initiated PCR Assay with Single-Copy Sensitivity for Human Immunodeficiency Virus Type 1 RNA in Plasma", JOURNAL OF CLINICAL MICROBIOLOGY, vol. 41, no. 10, 1 October 2003 (2003-10-01), pages 4531-4536, XP055207469, ISSN: 0095-1137, DOI: 10.1128/JCM.41.10.4531-4536.2003
- BLACK J B ET AL: "Frequent isolation of human herpesvirus 7 from saliva", VIRUS RESEARCH, AMSTERDAM, NL, vol. 29, no. 1, 1 July 1993 (1993-07-01), pages 91-98, XP023707472, ISSN: 0168-1702, DOI: 10.1016/0168-1702(93)90128-A [retrieved on 1993-07-01]
- C. BAGUTTI ET AL: "Detection of adeno- and lentiviral (HIV1) contaminations on laboratory surfaces as a tool for the surveillance of biosafety standards", JOURNAL OF APPLIED MICROBIOLOGY, vol. 111, no. 1, 20 May 2011 (2011-05-20), pages 70-82, XP055207479, ISSN: 1364-5072, DOI: 10.1111/j.1365-2672.2011.05042.x
- ISHII-WATABE AKIKO ET AL: "Detection of replication-competent adenoviruses spiked into recombinant adenovirus vector products by infectivity PCR", MOLECULAR THERAPY, NATURE PUBLISHING GROUP, GB, vol. 8, no. 6, 1 December 2003 (2003-12-01), pages 1009-1016, XP002495208, ISSN: 1525-0016, DOI: 10.1016/J.YMTHE.2003.09.001
- R. A. RODRIGUEZ ET AL: "Application of PCR-Based Methods To Assess the Infectivity of Enteric Viruses in Environmental Samples", APPLIED AND ENVIRONMENTAL MICROBIOLOGY, vol. 75, no. 2, 14 November 2008 (2008-11-14), pages 297-307, XP055207482, ISSN: 0099-2240, DOI: 10.1128/AEM.01150-08
- KALLE SAKSELA ET AL.: 'Human immunodeficiency virus type 1 mRNA expression in peripheral blood cells predicts disease progression independently of the numbers of CD4+ lymphocytes.' PROC. NATL. ACAD. SCI. USA vol. 91, 1994, pages 1104 - 1108, XP055164121
- F. REGGETI ET AL.: 'CD134 and CXCR4 expression corresponds to feline immunodeficiency virus infection of lymphocytes, macrophages and dendritic cells' JOURNAL OF GENERAL VIROLOGY vol. 89, 2008, pages 277 - 287, XP055164123
- M. Cabrerizo ET AL: "In vitro infection of human peripheral blood mononuclear cells by a defective hepatitis B virus with a deletion in the PreS1 region of the viral genome", JOURNAL OF VIRAL HEPATITIS., vol. 9, no. 4, 1 July 2002 (2002-07-01), pages 265-271, XP055286051, OXFORD, UK ISSN: 1352-0504, DOI: 10.1046/j.1365-2893.2002.00367.x
- Robert E. Lanford ET AL: "Persistence of extrahepatic hepatitis B virus DNA in the absence of detectable hepatic replication in patients with baboon liver transplants", JOURNAL OF MEDICAL VIROLOGY, vol. 46, no. 3, 1 July 1995 (1995-07-01), pages 207-212, XP055286064, US ISSN: 0146-6615, DOI: 10.1002/jmv.1890460307
- MORRIS T ET AL: "RAPID REVERSE TRANSCRIPTION-PCR DETECTION OF HEPATITIS C VIRUS RNA IN SERUM BY USING THE TAQMAN FLUOROGENIC DETECTION SYSTEM", JOURNAL OF CLINICAL MICROBIOLOGY, AMERICAN SOCIETY FOR MICROBIOLOGY, US, vol. 34, no. 12, 1 December 1996 (1996-12-01), pages 2933-2936, XP001052728, ISSN: 0095-1137
- Volker Meier ET AL: "HCV-RNA positivity in peripheral blood mononuclear cells of patients with chronic HCV infection: does it really mean viral replication?", World J. Gastroentero, 1 April 2001 (2001-04-01), XP055286081, Retrieved from the Internet: URL:http://www.wjgnet.com/1007-9327/full/v 7/i2/228.htm [retrieved on 2016-07-05]

## Description

**The invention relates to the methods** of detection of viruses with lymphotropism properties in biological substrates with low concentration of viral particles, evaluation of their viability and elimination of false-negative results of EIA and PCR test and may be used in the medical industry and biotechnology.

The detection of virus in biological substrates by its isolation in cell culture is a well-known method. Viruses isolated by cell culture method are identified by haemadsorption, hemagglutination or indirect immunofluorescence methods. Proper sampling and short-time transportation to the laboratory venue on appropriate media are essential for effective isolation of virus isolation in culture. It preserves virus viability and restricts bacteria and fungi reproduction (Laboratory diagnostic of virus infections, Online: http://www.center-hc.ru/). Many viruses, in particular hepatitis B virus (HBV), hepatitis C virus (HCV) and human immunodeficiency virus (HIV), are anthroponotic viruses affecting human cells only thus causing disease of human solely. There are no experimental models of these infections. Also, there are no cultivated cell cultures, particularly in the republic of Uzbekistan, on which one may adequately study cytopathogenic properties and viability of these viruses *in vitro.* Moreover, because of its complexity the method of isolation of viruses on cell cultures is not used for diagnostic purposes.

The immunological method for detection of viruses in biological material is known as enzyme-linked immunosorbent assay (ELISA) is based on using of specific viral proteins extracted from infected cells or produced by genetic engineering by the detection of antibodies to the number of virus antigens (Ilyina, E.N. et. al. Chronic virus liver diseases "Khronicheskiye virusnye zabolevaniya pecheni" Methodological manual for physicians "Metodicheskoye posobiye dlya vrachej" Moscow, 2001, p. 7-11).

In some virus infections, e.g. HCV, EIA detects the antibodies only, thus substantially restricting the evaluation capability of the progress and activity of infection. Moreover, EIA has sensitivity threshold, below which the detection of virus is impossible.

There are known methods of detection of viruses with lymphotropism properties in the biological materials, virus viability assessment and the exclusion of false-negative results of EIA and PCR is the detection of viral RNA or DNA by sampling of biological material and detection of presence of viral RNA or DNA by polymerase chain reaction (PCR) (Ilyina, E.N. et. al. Chronic virus liver diseases "Khronicheskiye virusnye zabolevaniya pecheni" Methodological manual for physicians "Metodicheskoye posobiye dlya vrachej" Moscow, 2001, p. 7-11).

The method relates to direct methods of the detection of the pathogen in the biological material thus permitting to evaluate the activity of viral process. Positive PCR-reaction confirms the presence of the virus in the liver and in the blood with high probability. PCR of the biological samples (plasma or blood proteins, tissue or organs' biopsy materials) not always allow detecting infection caused by viruses with lymphotropism properties, though such viruses may persist in substantially high concentrations in the lymphoid tissue (false-negative results of PCR) and *vice versa* positive PCR may be obtained without persistence of viruses. Furthermore, PCR has the sensitivity threshold, below which virus presence is not detectable by this method.

The closest to the proposed invention is a method of determining the pathogenicity of HIV by means of APOBEC3G degradation (Patent Application US 2009/0176202 A1). This method provides ways for analysis of APOBEC3G degradation, the study of cell lines for analysis of APOBEC3G degradation, as well as the detection of APOBEC3G degradation inhibitors. The method comprises growing cell lines that are producers of viral particles with the addition of inhibitors and without them. Next, the amount of APOBEC3G degradation in HIV viral particles is determined. Infestation of the cell cultures is then carried out, incubated cultured, after which quantity of viral nucleic acids is comparatively detected. Next, the number of virus particles are determined and they are compared with each other.

One more aspect of this disclosure is in its Fig. 14, according to which, in order to identify candidate compounds for inhibitors of HIV infectivity, T cells are infected with the HIV, and their cDNA level quantitatively detected. Prior to infection, viruses are treated with DNase.

The goal of present invention is increasing of reliability of infection detection caused by viruses with lymphotropism properties in a biological material.

The assigned goal is being resolved by the evaluation of viability of the viruses with lymphotropism properties by sampling of the biological material, detection of presence of viral RNA or DNA by the polymerase chain reaction (PCR); additionally lymphocytes suspension is obtained from the healthy human blood and the equal amount of biological material is added; the mixture is stirred, and incubated at 37°C for 6-8 hours; washing-out of lymphocytes from the plasma and the lymphocytes is being destructed; lymphocytes' cytoplasm is subjected for PCR test. The detection of viral RNA or DNA in the cytoplasm of lymphocytes indicates preserved viability of viruses; the absence of viral RNA or DNA viruses in the cytoplasm of lymphocytes indicated inactivation of viruses. Plasma or blood serum, biopsy samples of tissue or organs, the washouts from the medical instruments may be used as the biological samples. This method allows assessment of the viability of HBV, HCV or HIV viruses.

It is known that many viruses particularly those of hepatitis B virus (HBV), hepatitis C virus (HCV) and human immunodeficiency virus (HIV) can replicate in mononuclear blood cells, particularly, in the lymphocytes and the macrophages. It is known, that under HBV- and HCV-infections simultaneously cause inflammatory processes in the liver with all subsequent hepatitis, as well as secondary immunodeficiency with various degrees of T- lymphopenia and B-lymphopenia, imbalance of regulatory subpopulations of T-lymphocytes (T-helpers and T-suppressors), reduction of immune regulatory index (IRI) and dysgammaglobulinemia. The degree and grade of immunodeficiency has no relation with the degree of the pathologic process in the liver. Patients with chronic HBV and HCV infections have different intensity of pathological process in the liver tissue after some time, from weak to expressed, but regardless of this, stable and steady aggravation of secondary immunodeficiency.

The dissociation of degree of liver tissue injury and degree of secondary immunodeficiency in various nosological forms of chronic virus hepatitis supported the idea that the hepatitis and secondary immunodeficiency in HBV and HCV infections are associated, mutually aggravating, but not mutually conditional: that is, HBV and HCV along with hepatotropic property possess expressed lymphotropic property - direct property to cause secondary immunodeficiency. Thr differences in clinical appearances of the liver tissue injuries and degree of immunodeficiency in HBV and HCV infections are due to differences in degree of hepatotropic and lymphotropic properties of these viruses. Namely the differences in degrees of hepatotropic and lymphotropic properties of viruses determine the differences of the pathogenesis, clinical appearances and the pattern of antiviral therapy effect in chronic HBV and HCV infections in various stages of these diseases.

The identity of the lymphotropic properties of HBV and HCV, and HIV besides secondary immunodeficiency forming is confirmed also by commonality of their epidemiological features, mechanism of transfer, the progress of associated opportunistic infection (frequent respiratory diseases, intestinal infections), and particularly the development of the lymphogranulomatosis in different tissues of the organism. The development of lymphoid follicles' clusters, which is the lymphogranulomatosis, in various organs and tissues of the organism is supposed to be intrinsic for viral infections of the lymphoid cell system.

Considering lymphotropic properties of HBV, that regardless of serum titer, HBV can permanently persist in essentially high concentrations in the cytoplasm of lymphoid elements. This phenomenon is used in this method for reliable increasing and elimination of false-negative results by EIA and PCR, and detection of lymphotropic viruses in the biological material with concentration of viral particles below the threshold of test-sensitivity of EIA or PCR.

We used the lymphotropic properties of HBV, HCV and HIV in this evaluation method of virus viability - the ability of these viruses to penetrate and persist intracellularly in the healthy human lymphocytes during their *in vitro* incubation.

The evaluation of viability of viruses with lymphotropism properties, in particular HBV, HCV and HIV, is required after long term storage of viruses, for the control of the antiviral efficiency of various disinfecting chemical and physical factors against these viruses, as well as for the control of antiviral therapy.

Below is a description of the method for evaluation of viability of viruses with lymphotropism properties according to claim 1.

### I. Producing of suspension of viruses with lymphotropism properties

For producing of suspension of viruses, the biological material (plasma or blood serum, biopsy samples of tissue or organs, the wash-outs from medical instruments) is obtained. Then the biological material is subjected to quantitative PCR for the verification for the presence of viruses with lymphotropism properties and quantification of titer of viruses. Virus comprising biological material is kept in the frozen state in the refrigerator at below - 25°C temperature.

### II. Producing of lymphocytes suspension from healthy human subject:

a) healthy volunteers are tested for infection with lymphotropic viruses by EIA. Lymphocytes from healthy people with a negative result for study viruses are used in investigations;
b) to receive a sufficient amount of lymphocyte the blood is taken from ulnar vein in an amount of 20-30 ml in the morning from fasting healthy human subject. Then the blood per 7-8 ml is transferred to the centrifuge tubes containing 2 ml normal saline and 3 heparin drops ("Heparin" concentration of 5000 ME/ml; 3 drops contain 750 ME/ml of heparin). The resulting solution is stirred thoroughly;
c) the lymphocytes are separated from the whole heparin containing blood in ficoll-verografin gradient with density d=1.077 g/ml according to previously described method (Garib, F.Yu., Gurary, N.I., Garib, V.F. "Sposob opredeleniya subpopulyatsij limfotsitov (Determination method of lymphocytes' subpopulations)" // Rasmij Akhborotnoma. Tashkent, 1995, #1, p. 90 - UZ DP 2426). 2 ml of ficoll-verografin gradient is poured into the clean centrifuge tube, then the heparinized blood lays on its surface and the tube is centrifuged at 1500 RPM for 20 minutes. During centrifugation all blood cells, excluding lymphocytes, penetrate through ficoll-verografin gradient. Blood plasma remains above the gradient. In the border of ficoll-verografin gradient and plasma peculiar turbid ring consisting from pure lymphocytes is formed. The ring with lymphocytes is carefully pumped with a pipette and transferred to the clean centrifugal tube;
d) the lymphocytes are washed-out with 10 ml of normal saline 2-3 times with further centrifugation at 1500 RPM for 20 minutes.
e) after last centrifugation the supernatant is removed. The sediment containing lymphocytes is diluted and re-suspended in 600 µl of normal saline. Lymphocytes suspension may be stored no more than 1 day at temperature + 4°C.

### III. Evaluation of viruses with lymphotropism properties to penetrate and persist intracellularly in the human lymphocytes in vitro.

1) Biological material containing viruses with lymphotropism properties is taken from refrigerator and thawed at room temperature.
2) equal amounts (300 µl) of virus containing biological material and the suspension of healthy human lymphocytes is transferred with pipette to the clean centrifugal tube; the contents mixed and placed for the incubation (incubation of viruses with lymphocytes *in vitro*) into the thermostat at +37°C for 6-8 hours. The testing tube is mixed with shacking every 1.5-2 hours.
3) The washing-out of lymphocytes. Testing tube is removed from the thermostat. 6-8 ml of normal saline is added, mixed and centrifuged at 1500 RPM for 20 minutes. The lymphocytes are sediment on the bottom of the tube. The supernatant (mixture of plasma with saline) is entirely removed. The lymphocytes are washed out in normal saline and sediment 2-3 times. After last centrifugation, the supernatant is removed the suspension of the lymphocytes (sediment) is diluted with 500 µl of normal saline and transferred to plastic 1.5 lock tube (Eppendorf tube).
4) Thereafter the tube is placed into the freezer of house grade refrigerator overnight. The lymphocytes are destructed under slow freezing condition.
5) The removal of the membrane of destroyed lymphocytes. Next day the tubes from the freezer thawed at room temperature. Then the membranes of destroyed lymphocytes are removed by centrifugation at 3000 RPM for 30 minutes. Membranes are precipitated on the bottom of the tube and lymphocytes' cytoplasm content remains in the supernatant.
6) The supernatant from the tube is transferred and subjected to quantitative PCR of viral RNA or DNA in the cytoplasm of lymphocytes that previously was in infected patient's plasma.

### IV. Assessment of results.

1. Positive PCR for the presence of viral RNA or DNA in the cytoplasm of lymphocytes indicates the remaining virus viability, i.e. ability to penetrate and persist in human lymphocytes *in vitro.*
2. Negative PCR for the presence of viral RNA or DNA in the cytoplasm of lymphocytes indicates the loss (inactivation) of virus viability, i.e. loss of their ability to penetrate and persist inhuman lymphocytes *in vitro.*

Claimed methods are certified by the following examples:

### Example #1.

### The assessment of viability of viruses with lymphotropism properties

The blood is obtained from ulnar vein from the patient after receiving antiviral therapy for hepatitis C. The plasma separated from whole blood and subjected to the quantitative PCR for the verification of HCV presence and quantification virus titer. The PCR is negative. Tested plasma is kept in the freezer at below -25°C temperature.

Simultaneously the 20-30 ml of blood from healthy human volunteers is obtained in the morning from ulnar vein. The part of blood plasma is subjected to PCR for viruses with lymphotropism properties infection. The lymphocytes from the healthy humans with negative results of testing for infection are used for further investigation. Then the 7-8 ml blood aliquots transferred to the centrifuge tubes containing 2 ml of normal saline and 3 heparin drops ("Heparin" concentration is 5000 ME/ml, 3 drops contain 750 ME/ml of heparin). The solution in the tube is mixed thoroughly. The lymphocytes separated from the whole heparinized blood in ficoll-verografin gradient with d=1.077 g/ml density according to Garib, F.Yu. *et al* method. 2 ml the ficoll-verografin gradient poured into clean centrifuge tube, heparinized blood lays on the surface of gradient and centrifuged at 1500 RPM for 20 minutes. All blood cells excluding lymphocytes penetrate the ficoll-verografin gradient and sediment under it. The blood plasma located above the gradient. On the border between ficoll-verografin gradient and plasma, the peculiar turbid ring with pure lymphocytes suspension is formed. The ring with lymphocytes carefully sucked with a pipette and transferred to the clean centrifuge tube. The lymphocytes are washed out in normal saline and sediment 2-3 times. After last centrifugation, the supernatant is removed. The sediment containing lymphocytes is diluted with 600 µl saline and re-suspended. The lymphocytes suspension may be stored no more than 1 day at + 4°C temperature.

The testing plasma from the freezer is thawed at room temperature. Equal volumes (300 µl) of plasma and the suspension of lymphocytes are transferred to clean centrifuge tube with pipette, mixed and placed for incubation in thermostat at +37°C temperature for 6-8 hours. The tube is mixed by shaking every 1.5-2 hours.

After incubation the tube is removed from the thermostat. 6-8 ml saline is added, mixed and centrifuged at 1500 RPM for 20 minutes. The lymphocytes sediment on the bottom of the tube. The supernatant (mixture of plasma and normal saline) is removed. Then 2-3 times wash-out in normal saline and the lymphocytes sedimentation is performed *ditto.* After last centrifugation, the supernatant is removed, and suspension of lymphocytes (sediment) is diluted by adding 300 µl of normal saline, and transferred to the 1.5 ml lock tube (Eppendorf tube).

Thereafter lymphocyte membranes are destructed by overnight placing into house grade freezer. Next day the tubes are thawed at the room temperature. Then the membranes of destroyed lymphocytes are removed from suspension by centrifugation of tube at 3000 RPM for 30 minutes. The membranes are precipitated on the bottom of the tubes and lymphocyte cytoplasm contents remain in the supernatant. The supernatant is transferred from the tube and subjected to quantitative PCR for the presence of HCV virus in the cytoplasm of lymphocytes. Positive PCR test for HCV indicates the preservation of the HCM viability and the requirement of further antiviral therapy.

### Example #2.

The liver tissue sampled by liver puncture from a patient, who was given antiviral therapy for hepatitis B. Liver biopsy sample is homogenized in 1.5 ml normal saline; transferred to the centrifuge tube, centrifuged at 1500 RPM for 20 minutes; and the supernatant transferred to the tube. One part of supernatant is subjected to quantitative PCR testing for the presence of HCV virus and quantification of virus titer. PCR test for HCV is positive. The biopsy sample is kept in the freezer in the refrigerator at below -25°C temperature.

The lymphocyte suspension from healthy human is made as described in example #1. The supernatant from liver biopsy sample homogenate is thawed at room temperature. The equal volumes (300 µl) of the supernatant and lymphocyte suspension is added to the tube by automatic pipette; the resulting solution is mixed and placed for incubation into thermostat at +37°C temperature for 6-8 hours. Testing tube is mixed by shaking every 1.5-2 hours.

The tube is removed from thermostat. 6-8 ml of normal saline is added, mixed and centrifuged at 1500 RPM for 20 minutes. The lymphocytes sediment on the bottom of the tube. The supernatant (mixture of plasma with normal saline) is removed entirely. The supernatant (mixture of plasma and normal saline) is removed. Then 2-3 times wash-out in normal saline and the lymphocytes sedimentation is performed *ditto.* After last centrifugation, the supernatant is removed and suspension of lymphocytes (sediment) is diluted by adding 300 µl of normal saline. Thereafter the destruction of lymphocyte membranes is performed by putting testing tube into house grade freezer overnight.

Thereafter lymphocyte membranes are destructed by overnight placing into house grade freezer. Next day the tubes are thawed at the room temperature. Then the membranes of destroyed lymphocytes are removed from suspension by centrifugation of tube at 3000 RPM for 30 minutes. The membranes are precipitated on the bottom of the tubes and lymphocyte cytoplasm contents remain in the supernatant. The supernatant is transferred from the tube and subjected to quantitative PCR for the presence of HBV virus in the cytoplasm of lymphocytes. Negative PCR for HBV indicates the lost virus viability (inactivation).

### Illustrative Example #3.

The detection of viruses with lymphotropism properties in biological material with the concentration of virus below EIA and PCR sensitivity threshold.

In blood center, the blood plasma from 6-8 ml of blood is being tested for viruses with lymphotropism properties. One part of plasma is subjected to quantitative PCR for testing the presence of HBV, HCV or HIV viruses and the quantification of virus titer. PCR for the presence of viruses is negative. Tested plasma is stored in the freezer at below -25°C.

The lymphocyte suspension from healthy human subject is performed as described in example #1.

The testing plasma from the freezer is thawed at room temperature. Equal volumes (300 µl) of plasma and the suspension of lymphocytes is transferred to clean centrifuge tube with automatic pipette, mixed and placed for incubation in thermostat at +37°C for 6-8 hours. The tube is mixed by shaking every 1.5-2 hours.

The tube is removed from thermostat. 6-8 ml of normal saline is added, mixed and centrifuged at 1500 RPM for 20 minutes. The lymphocytes sediment on the bottom of the tube. The supernatant (mixture of plasma with normal saline) is removed entirely. The supernatant (mixture of plasma and normal saline) is removed. Then 2-3 times wash-out in normal saline and the lymphocytes sedimentation is performed *ditto.* After last centrifugation, the supernatant is removed and suspension of lymphocytes (sediment) is diluted by adding 300 µl of normal saline.

Thereafter lymphocyte membranes are destructed by overnight placing into house grade freezer.

Next day the tubes are thawed at the room temperature. Then the membranes of destroyed lymphocytes are removed from suspension by centrifugation of tube at 3000 RPM for 30 minutes. The membranes are precipitated on the bottom of the tubes and lymphocyte cytoplasm contents remain in the supernatant. The supernatant is transferred from the tube and subjected to quantitative PCR for detection of HBV, HCV and HIV viruses in the content of lymphocytes' cytoplasm. Positive PCR for HCV indicates the presence of HCV virus in the donor plasma its ineligibility for the transfusion.

### Illustrative Example #4.

The elimination of EIA and PCR false-negative results.

The 6-8 ml of blood obtained in the morning from fasting donor from the ulnar vein. Whole blood transferred to the tube, subjected to the sedimentation, and the serum is obtained; one part of serum is subjected to PCR for the presence of HBV, HCV or HIV viruses and quantification of virus titer. The PCR tests are negative. The rest of the blood serum is stored in the tube. The lymphocyte suspension from healthy human subject is performed as described in example #1.

The lymphocytes separated and destructed by overnight freezing in house grade refrigerator. Next day the tube is thawed at room temperature. Then the membranes of destroyed lymphocytes are removed from suspension by centrifugation at 3000 RPM for 30 minutes. The membranes precipitate on the bottom of the tube, and lymphocyte cytoplasm contents remain in the supernatant. The supernatant is transferred from the tube and subjected to quantitative PCR for detection of HBV, HCV and HIV viruses in the cytoplasm of lymphocytes. Positive PCR for HBV indicated the presence of HBV in the donor blood.

In standard PCR, the detection rate of HBV and HCV in the Republic is 2.7%. According to the epidemiological data new cases of hepatitis B (HBV) and hepatitis C (HCV) transfer occur due to the transfusion of infected blood or its components in 2.2%-5.6%. To reveal the reasons behind and elimination of HBV infection in recipients the lymphotropic properties of the virus were used.

The serums from 309 donor blood samples were tested by PCR for HBV markers detection rate.

PCR revealed HBV in 6 out of 209 serum samples that estimated 1.94% of all number of donors' sample. The same PCR study (study of lymphocytes content from the same donors) revealed HBV in 17 out of 309 samples, estimated 7.44% of all donors' samples. Thus, the standard PCR testing of blood serum was false-negative in 5.50% of samples, thus being the reason of HBV infection in recipients by transfusion of infected blood or it components.

The implementation of PCR testing of lymphocytes for detection of viruses in blood centers will allow significantly reduction of frequency or eliminate the cases of HBV, HCV and HIV after transfusion of blood or it components.

## Claims

1. A method for evaluating the viability of viruses with lymphotropism properties in patients after testing for lymphotropic virus infection, comprising:
testing, by subjecting a biological material that has been collected from a patient suspected of infection by a lymphotropic virus, to polymerase chain reaction, PCR, wherein said testing indicates no infection by a lymphotropic virus,
**characterized by**:
obtaining a lymphocyte suspension from a blood sample of at least one healthy human subject;
combining said lymphocyte suspension with an equal amount of said biological material to form an admixture;
incubating the admixture;
washing-out the lymphocytes from said admixture;
removing lymphocyte membranes, separating the lymphocyte cytoplasm content suspected of infection by said lymphocyte virus by centrifugation; and
detecting the presence or absence of virus RNA or DNA in the lymphocyte cytoplasm content by PCR,
wherein the presence of the virus RNA or DNA in the lymphocyte cytoplasm content, with the earlier non-detection of infection, indicates the preserved viability of viruses in said biological material of said patient, and
wherein the absence of the virus RNA or DNA in the lymphocyte cytoplasm content indicates the loss of virus viability in said biological material of said patient.

2. The method according to claim 1, wherein said biological material is selected from the group consisting of plasma, blood serum, biopsy tissues or organs, medical instrument wash-outs.

3. The method according to claim 1, wherein the virus RNA or DNA in said biological material of said patient suspected of infection is selected from the group consisting of hepatitis B (HBV), hepatitis C (HCV), human immunodeficiency (HIV).

4. The method according to claim 1, wherein, in said incubating, said admixture is incubated at about 37°C for about 6-8 hours.

5. The method according to claim 1, wherein the viability of said virus RNA or DNA is the ability of the virus RNA or DNA to penetrate and persist intracellularly in the lymphocytes of at least one healthy human subject during in vitro incubation.

6. The method according to claim 1, wherein said at least one healthy human subject is negative for the presence of the virus RNA or DNA.

7. The method according to claim 1, wherein, in said step of removing, lymphocytes within said admixture are destroyed.

## Patentansprüche

1. Verfahren zur Beurteilung der Viabilität von Viren mit Lymphotropismus-Eigenschaften in Patienten nach Test auf Infektion mit lymphotropen Viren, beinhaltend:
Testen, durch Unterziehen von von einem Patienten erhaltenem biologischem Material, bei welchem Patient der Verdacht einer Infektion mit einem lymphotropen Virus besteht, der Polymerase-Kettenreaktion, PCR, wobei das Testen keine Infektion durch ein lymphotropes Virus anzeigt,
**gekennzeichnet durch**:
Erhalten einer Lymphozytensuspension von einer Blutprobe von wenigstens einer gesunden Person;
Zusammenfügen der Lymphozytensuspension mit einer gleichen Menge des biologischen Materials zum Bilden einer Mischung;
Inkubieren der Mischung;
Auswaschen der Lymphozyten aus der Mischung;
Entfernen von Lymphozytenmembranen, Abtrennen des der Infektion mit dem Lymphozyten-Virus verdächtigten Lymphozyten-Zytoplasmainhalts durch Zentrifugieren; und
Erfassen der Anwesenheit bzw. Abwesenheit von viraler RNA bzw. DNA in dem Lymphozyten-Zytoplasmainhalts durch PCR,
wobei die Anwesenheit von viraler RNA bzw. DNA in dem Lymphozyten-Zytoplasmainhalt, trotz der früheren Nicht-Erfassung einer Infektion, anzeigt, dass die Viabilität von Viren in dem biologischen Material des Patienten fortbesteht, und wobei die Abwesenheit der viralen RNA bzw. DNA in dem Lymphozyten-Zytoplasmainhalt den Verlust an Virus-Viabilität in dem biologischen Material des Patienten anzeigt.

2. Verfahren gemäß Anspruch 1, wobei das biologische Material Plasma, Blutserum, Biopsiegewebe oder -organe oder Waschflüssigkeit von medizinischen Instrumenten beinhaltet.

3. Verfahren gemäß Anspruch 1, wobei die virale RNA bzw. DNA in dem biologischen Material des der Infektion verdächtigen Patienten die von Hepatitis B (HBV), Hepatitis C (HCV) oder Humane Immunschwäche (HIV) ist.

4. Verfahren gemäß Anspruch 1, wobei, beim Inkubieren, die Mischung für etwa 6 bis 8 Stunden bei etwa 37°C inkubiert wird.

5. Verfahren gemäß Anspruch 1, wobei die Viabilität der viralen RNA bzw. DNA die Fähigkeit der viralen RNA bzw. DNA ist, während der In-vitro-Inkubation in die Lymphozyten wenigstens einer gesunden Person einzudringen und intrazellulär darin zu persistieren.

6. Verfahren gemäß Anspruch 1, wobei die wenigstens eine gesunde Person negativ für die Anwesenheit der viralen RNA bzw. DNA ist.

7. Verfahren gemäß Anspruch 1, wobei, bei dem Schritt des Entfernens, Lymphozyten in der Mischung zerstört werden.

## Revendications

1. Procédé d'évaluation de la viabilité de virus avec des propriétés de lymphotropisme chez des patients après un test de recherche d'infection par un virus lymphotrope, comprenant :
le test, par soumission de matériel biologique qui a été collecté chez un patient suspecté d'infection par un virus lymphotrope, à une réaction d'amplification en chaîne par polymérase, PCR, dans lequel ledit test indique l'absence d'infection par un virus lymphotrope, **caractérisé par** :
l'obtention d'une suspension de lymphocytes à partir d'un échantillon de sang d'au moins un sujet humain sain ;
la combinaison de ladite suspension de lymphocytes avec une quantité égale dudit matériel biologique pour former un mélange ;
l'incubation du mélange ;
l'élimination par lavage des lymphocytes dudit mélange ;
le retrait des membranes lymphocytaires, la séparation du contenu cytoplasmique lymphocytaire suspecté d'infection par ledit virus lymphocytaire par centrifugation ; et
la détection de la présence ou de l'absence d'ARN ou d'ADN viral dans le contenu cytoplasmique lymphocytaire par PCR,
dans lequel la présence du virus à ARN ou à ADN dans le contenu cytoplasmique lymphocytaire, avec la non détection d'infection préalable, indique la viabilité préservée de virus dans ledit matériel biologique dudit patient, et
dans lequel l'absence de l'ARN ou de l'ADN viral dans le contenu cytoplasmique lymphocytaire indique la perte de viabilité du virus dans ledit matériel biologique dudit patient.

2. Procédé selon la revendication 1, dans lequel ledit matériel biologique est sélectionné dans le groupe constitué par le plasma, le sérum sanguin, les tissus et organes de biopsie, les liquides de rinçage d'instruments médicaux.

3. Procédé selon la revendication 1, dans lequel l'ARN ou l'ADN viral dans ledit matériel biologique dudit patient suspecté d'infection est sélectionné dans le groupe constitué par l'hépatite B (HBV), l'hépatite C (HCV), l'immunodéficience humaine (VIH).

4. Procédé selon la revendication 1, dans lequel dans ladite incubation, ledit mélange est incubé à environ 37 °C pendant environ 6 à 8 heures.

5. Procédé selon la revendication 1, dans lequel la viabilité dudit ARN ou ADN viral est la capacité de l'ARN ou ADN viral à pénétrer et à persister intracellulairement dans les lymphocytes d'au moins un sujet humain sain durant l'incubation *in vitro.*

6. Procédé selon la revendication 1, dans lequel ledit au moins un sujet humain sain est négatif pour la présence d'ARN ou d'ADN viral.

7. Procédé selon la revendication 1, dans lequel à ladite étape de retrait, les lymphocytes à l'intérieur du mélange sont détruits.
